Europäisches Patentamt

(19) European Patent Office

Office européen des brevets

(11) Publication number: **0 271 623**
**B1**

(12) # EUROPEAN PATENT SPECIFICATION

(45) Date of publication of the patent specification:
07.03.90

(51) Int. Cl.⁴: **C07C 37/08**

(21) Application number: **86309978.4**

(22) Date of filing: **19.12.86**

(54) **Process for producing hydroquinone.**

(43) Date of publication of application:
**22.06.88 Bulletin 88/25**

(45) Publication of the grant of the patent:
**07.03.90 Bulletin 90/10**

(84) Designated Contracting States:
**AT BE CH DE ES FR GB GR IT LI LU NL SE**

(56) References cited:
**EP-A- 0 030 088**
**US-A- 3 978 142**

**CHEMICAL ENGINEERING, vol. 32, no. 12, 9th
June 1975, pages 50-51; A.H. OLZINGER: "New route to
hydroquinone"**

(73) Proprietor: **NESTE OY, Kellaniemi,
SF-02150 Espoo 15(FI)**

(72) Inventor: **Koskimies, Salme, Palopirtintie 17 A 7,
SF-00930 Helsinki(FI)**
Inventor: **Haimela, Taru, Leppapolku 3,
SF-04600 Mantsala(FI)**

(74) Representative: **Lamb, John Baxter et al, MARKS &
CLERK 57/60 Lincoln's Inn Fields, London
WC2A 3LS(GB)**

ACTORUM AG

**Description**

The present invention concerns a process for producing hydroquinone by oxidizing a starting material mixture consisting of a component containing tertiary alkylaromatic and a component containing hydroperoxides produced in the oxidizing reaction of the alkylaromatics, selectively with the aid of oxygen at temperature from 60 to 180°C, and by separating from the mixture the dihydroperoxide of p-diisopropylbenzene, which is decomposed by acid catalysis to become hydroquinone, at 20-150°C.

It is known in the art that aliphatic hydrocarbons, such as isobutane, 2-methylbutane, decahydronaphthalene, and alkyl aromatics like cumene, methylnaphthalene, diisopropylbenzene, etc. can be oxidized with the aid of oxygen to become equivalent hydroperodixes so that either the tertiary or -aromatic carbon reacts. It is equally known in the art that said hydroperoxides react under effect of acid catalysts, producing the corresponding phenol, or alcohol, and aldehyde or ketone.

Said process has, as known in the art, been used for instance in producing phenol from cumene, cresols from cymene, or resorcinol from m-diisopropylbenzene and hydroquinone from p-diisopropylbenzene. In procedures of prior art, resorcinol and hydroquinone have been produced using pure, relatively expensive m- or p-diisopropylbenzenes as starting materials.

The process of the present invention for producing hydroquinone selectively from the hydrocarbon mixture obtained as a by-product of cumene is characterized in that for said component containing tertiary alkylaromatic is used a product obtained as a by-product in producing cumene which has boiling range 170-220°C and which contains diisopropylbenzenes from 10 to 80% by weight and mixture of $C_4$-$C_9$ alkylbenzenes from 20 to 90% by weight, preferably from 20 to 60% by weight, in particular t-butylbenzene, 1,1-dimethylpropylbenzene, i- and 1-hexylbenzenes, and 2- and 3-phenylhexanes, whereby after oxidation, by cooling the reaction mixture, dihydroperoxide of p-diisopropylbenzene will selectively crystallize, the latter being separated and decomposed by acid catalysis to become hydroquinone and acetone, while the monohydroperoxides and the dihydroperoxide of m-diisopropylbenzene remain in the oxidizing solution and are returned to the oxidizing reactor.

The process of the invention is based on the observation that in the oxygen oxidizing process, the reactivities of different alkylaromatics and the solubilities of the oxidation products obtained are different. This has further opened up the possibility that alkylaromatic mixtures may also be used for starting material and that it is then possible to optimate the reaction conditions so that only certain compounds, in the first place tertiary aralkyl compounds, are oxidized. If in addition for starting material is used the hydrocarbon mixture obtained as by-product when producing cumene, the oxidation products that are obtained differ so much in solubility from the mother solution that the dihydroperoxide of p-diisopropylbenzene can be selectively separated from the other oxidation products directly by crystallizing, whence further hydroquinone is obtained by acid-catalytic decomposition.

Acids known to be suitable include mineral acids, such as sulphuric acid, phosphoric acid, etc., acid aluminosilicates, sulphur trioxide, and Lewis acids, such as borotrifluoride, aluminium trichloride, etc. In the process of the invention, however, solid ion exchange resin containing acid groups is preferably used for decomposing the dihydroperoxides, whereby the advantage is gained, e.g. in comparison with sulphuric acid, that the extra neutralizing step is avoided because the catalyst can be removed by filtering. For solvent in the decomposing step one may use ketones, such as acetone, methylethylketone, methylisobutylketone, and/or low-boiling aliphatic or aromatic hydrocarbons.

It is thus understood that the process of the invention is based on the different reactivities in oxygen oxidation of the alkyl aromatics present in the by-product mixture obtained when producing cumene, and in particular on the different solubility of dihydroperoxide of p-diisopropylbenzene from that of the other oxidation products in the mother solution. This has further enabled the reaction conditions to be optimated so that only certain compounds, in the first place tertiary aralkyl compounds, are oxidized and that of the oxidation products only dihydroperoxide of p-diisopropylbenzene is precipitated. In these circumstances, the other hydrocarbon components in the reaction mixture act, in contrast, like an inert diluent. On one hand, they promote the separation in crystalline form of dihydroperoxide of p-diisoprpylbenzene that is formed, while on the other hand they cause retardation of the oxidizing reaction in oxidizing conditions typical to pure diisopropylbenzene.

Using in the oxidizing phase a temperature higher than normal, that is 60-180°C, preferably 90-130°C, the oxidizing may, however, be effected at a speed equivalent to that of the pure starting material components. Moreover, the number of by-products produced is then smaller in comparison with the oxidizing reaction carried out at a lower temperature.

It is known in the art that the oxygen oxidization process of diisopropylbenzene progresses in such manner that in the first step corresponding monohydroperoxides are formed, and when the content of monohydroperoxides in the solution has ben sufficiently elevated (over 10%) dihydroperoxides begins to form. It is moreover known in the art that the oxidation can be catalyzed by adding the corresponding dihydroperoxide to the starting material.

In the process of the invention, various hydroperoxides may also be used as catalysts. Highly useful with a view to producing dihydroperoxides is recirculation of oxidized mother solution from which the dihydroperoxide of p-diisopropylbenzene has been removed, back to the oxidizing reactor. This is primarily due to the fact that the mother solution contains remarkable quantities of monohydroperoxides of diisopropylbenzene and some m-diisopropylbenzene, and these may further act as catalysts of the oxidizing reaction. Endeavours are made to maintain the total hydroperoxide content in the oxygen oxidation process in the range from 10 to 80 per cent by weight, preferably in the range from 20 to 60 per cent by weight. The proportion of recirculated mother solution, related to the starting material, may be 0.01-10:1, preferably 0.05-2:1.

For separating the dihydroperoxides of diisopropylbenzene from the mother solution a number of methods are known to exist. The dihydroperoxides may for instance be extracted into a 4% aqueous NaOH solution, from which they may further be transferred into a methylisopropylketone solution for decomposing. Other procedures known in the art are the separation of dihydroperoxides using anion exchange resin containing basic groups, or crystallizing the dihydroperoxides from the mother solution either as sodium salts or by adding inert aliphatic or aromatic hydrocarbon to the mother solution.

In the process of the invention, in particular crystallization of dihydroperoxide of p-diisopropylbenzene from the mother solution is quite much easier than in the normal oxidizing process of diisopropylbenzenes because the starting material often already contains 50% hydrocarbons acting as inert agents. Furthermore, the process of the invention differs from processes of prior art in that dihydroperoxide of m-diisopropylbenzene, which is also produced in the oxidizing step, does not crystallize in the circumstances that are applied. The process is therefore selective in particular for separating dihydroperoxide of p-diisopropylbenzene, and this further renders it possible that the hydroquinone obtained on acid decomposition contains no significant amounts of phenolic impurities.

In the process of the invention, the by-product component produced in conjunction with the cumene-producing process, containing about 25% p-diisopropylbenzene, is oxidized at 60-180°C, preferably at 90-100°C, so that the total hydroperoxide content will be 10-80 per cent by weight, preferably 20-60 per cent by weight. The dihydroperoxide of p-diisopropylbenzene that is formed is crystallized from the mother solution, using cooling. The mother solution is recirculated to the oxidizing reactor so that its proportion related to the starting material will be 0.01-10:1, preferably 0.25-2:1. The crystalline dihydroperoxide of p-diisopropylbenzene is dissolved in methylisobutylketone and decomposed with the aid of an acid catalyst, preferably an acid ion exchange resin, to become hydroquinone and acetone at 20-150°C, preferably at 20-120°C.

The invention differs from the processes of prior art particularly in that it renders possible the production of hydroquinone selectively from the hydrocarbon mixture obtained as a by-product when producing cumene, said mixture being considerably more advantageous than pure p-diisopropylbenzene, which is used as starting material in the processes of prior art. Since application of the process implies no purifying of the by-product obtained in cumene production, and since the intermediate product, i.e., dihydroperoxide of p-diisopropylbenzene, when applying the process of the invention, is simply separable by crystallizing from the rest of the oxidation products, thus enabling production of hydroquinone which is free from other phenol components. Since in the process of the invention furthermore acid ion exchange resin is used for catalyst in the decomposing step, thus obviating the extra neutralizing step, the process may be considered quite simple indeed.

The invention is described more in detail in the attached, non-restrictive embodiment examples.

Examples 1-5

Into a cylindrical oxidizing reactor (diameter 45 mm and height 450 mm) provided with heating jacket, thermometer, oxygen dispersing tube and oxygen flow meter were added 500 ml of the hydrocarbon by-product obtained in cumene production, the composition of said by-product being presented in Table I below.

**Table I**

| Component | Content % by weight |
|---|---|
| Isopropylbenzene | 1.342 |
| Tertiary butylbenzene | 3.771 |
| Secondary butylbenzene | 0.9672 |
| 2-phenyl-3-methylbutane | 0.352 |
| 1,1-dimethylpropylbenzene | 2.608 |
| 1-methylbutylbenzene | 0.403 |
| 1,3-diisopropylbenzene | 21.379 |
| $i\text{-}C_6$-benzene | 7.606 |
| 1,4-diisopropylbenzene | 25.942 |
| 1,1-dimethylbutylbenzene | 8.647 |
| $1\text{-}C_8$-benzene | 1.579 |
| 3-phenylhexane | 5.810 |
| 2-phenylhexane | 2.245 |
| $C_7$-benzene | 0.491 |
| $C_9$-benzene | 0.611 |

The hydrocarbon mixture was heated to 100°C. Thereafter, oxygen was conducted into the reactor through the oxygen dispersing tube at constant flow rate 330 ml/min.

After oxidization had been carried on for 12.5 hrs, the total peroxide content of the solution was 1.1%. The reaction product was diluted with starting material so that the total hydroperoxide content in the mixture became 5.0%. This mixture was oxidized in 24.5 hrs so that the total hydroperoxide content became 14.4%.

In case the proportion of hydroperoxide catalyst in the starting material was 0.9 or 4.5 per cent by weight, or the reaction temperature was 90°C, the oxidization products were as presented in Table II.

**Table II**

| Ex-ample | Oxidizing time hours | Hydroperoxides per cent at beginning | at end | m-MHP[a] % | p-MHP[a] % | m-DHP[b] % | p-DHP[b] % | m-HHP[c] % | p-HHP[c] % |
|---|---|---|---|---|---|---|---|---|---|
| 1[d] | 12.5 | – | 1.1 | | | | | | |
| 2[d] | 24.5 | 0.5 | 14.4 | | | | | | |
| 3[d] | 26.0 | 0.9 | 20.0 | 5.3 | 9.4 | 1.9 | 0.9 | | |
| 4[d] | 22.0 | 4.5 | 35.8 | 6.4 | 12.8 | 2.5 | 2.3 | 2.7 | 4.5 |
| 5[e] | 40.0 | 1.3 | 8.5 | | | | | | |

(a) MHP = monohydroperoxide of diisopropylbenzene
(b) DHP = dihydroperoxide of diisopropylbenzene
(c) HHP = hydroxyhydroperoxide of diisopropylbenzene
(d) Oxidizing temperature 100°C
(e) Oxidizing temperature 90°C

### Examples 6-10

A hydrocarbon mixture, having the composition presented in Table I, was oxidized with oxygen in the manner described in Examples 1-5, at 110°C. For catalyzing the oxidizing reaction, partly oxidized mother solution was used, which was so circulated that the total hydroperoxide contents in the starting material were 0.9, 2.4, 4.9, 5.1 and 7.6%, respectively. The resulting oxidation products were as in Table III.

Table III

| Ex-ample | Oxidizing time hours | Hydroperoxides per cent | | m-MHP[a] % | p-MHP[a] % | m-DHP[b] % | p-DHP[b] % | m-HHP[c] % | p-HHP[c] % |
|---|---|---|---|---|---|---|---|---|---|
| | | at be-ginning | at end | | | | | | |
| 6 | 10.5 | 0.9 | 19.9 | | | | | | |
| 7 | 8.5 | 2.4 | 19.8 | | | | | | |
| 8 | 9.0 | 4.9 | 34.0 | 3.7 | 7.5 | 5.8 | 3.8 | 2.3 | 1.6 |
| 9 | 12.0 | 7.6 | 29.0 | 3.4 | 7.0 | 4.2 | 3.0 | 2.6 | 1.5 |
| 10 | 7.0 | 5.1 | 25.5 | | | | | | |

(a)–(c) as in Examples 1–5.

From the reaction mixtures described in Examples 4 and 8-10, a solid reaction product was precipitated by means of cooling, its composition being p DHP 65-75%, p HHP 20-30%, and m DHP 0-5%.

The precipitate was dissolved in methylisobutylketone (MiBK) to make approximately a 10% solution, and the hydroperoxides were decomposed either with sulphuric acid or using acid ion exchange resin (Amberlyst H+) to become corresponding phenols, in the manner described in Examples 11-15, the decomposing temperature being 60°C and time, 20 min.

Table IV

| Example (Sample)[a] | Decompos. catalyst | Starting material | | Product | |
|---|---|---|---|---|---|
| | | p-DHP % | m-DHP % | Hydroq., % Yield, %[b] | Res., % Yield, %[b] |
| 11 (4) | 0.15% $H_2SO_4$ | 4.26 | – | 2.08 (100.5%) | – |
| 12 (8) | 0.15% $H_2SO_4$ | 5.24 | – | 2.56 (100.8%) | – |
| 13 (9) | 0.15% $H_2SO_4$ | 4.65 | – | 2.27 (101.9%) | – |
| 14 (10) | 0.15% $H_2SO_4$ | 4.24 | 0.33 | 2.07 (100%) | 0.16 (99.5%) |
| 15 (10) | 4% Amberlyst | 4.24 | 0.33 | 2.01 (97%) | 0.15 (95%) |

(a) The precipitates obtained in Examples 4 and 8–10, dissolved in MiBK
(b) The yield has been calculated on the basis of the quantities of p-DHP and m-DHP from the result of analysis

## Claims

1. A process for producing hydroquinone by oxidizing a starting material mixture consisting of a component containing tertiary alkylaromatic and of a component containing hydroperoxides produced in the oxidation reaction of alkylaromatics selectively with the aid of oxygen at 60-180°C and by separating from the mixture the dihydroperoxide of p-diisopropylbenzene, which is decomposed by acid catalysis to become hydroquinone, at 20-150°C, characterized in that for said component containing tertiary alkylaromatic is used such product obtained as a by-product in producing cumene which has boiling range 170-220°C and contains 10-80 per cent by weight diisopropylbenzenes and 20-90 per cent by weight mixture of $C_4$-$C_9$ alkylbenzenes, whereby on cooling the reaction mix after oxidation dihydroperoxide of p-diisopropylbenzene crystallizes selectively, this being separated and decomposed by acid catalysis to become hydroquinone and acetone, while the monohydroperoxides and dihydroperoxide of m-diisopropylbenzene remain in the oxidizing solution and are returned to the oxidation reactor.

2. Process according to claim 1, characterized in that said component containing tertiary alkylaromatic contains diisopropylbenzenes 10-80 per cent by weight and mixture of $C_4$-$C_9$ alkylbenzenes, in particular t-butylbenzene, 1,1-dimethylpropylbenzene, i- and 1-hexylbenzenes, and 2- and 3-phenylhexanes, 20-60 per cent by weight.

3. Process according to claim 1 or 2, characterized in that the dihydroperoxide of p-diisopropylbenzene is decomposed to become hydroquinone with the aid of acid ion exchange resin.

4. Process according to any one of claims 1-3, characterized in that the proportion of oxidizing mixture returned to the oxidation reactor, of the quantity of starting material, is in the range 0.01-10:1, preferably 0.05-2:1.

**Patentansprüche**

1. Verfahren zur Produktion von Hydrochinon durch Oxidation einer Mischung von Ausgangsstoffen, die aus einer tertiäre alkylaromatische Verbindungen enthaltenden Komponente und einer Hydroperoxide enthaltenden Komponente besteht, wobei die Hydroperoxide selektiv in der Oxidationsreaktion von alkylaromatischen Verbindungen mittels Sauerstoff bei 60–180°C erzeugt werden, und durch Trennung des Dihydroperoxids von p-Diisopropylbenzol von der Mischung, welches Dihydroperoxid bei 20–150°C durch saure Katalyse zersetzt wird, um Hydrochinon zu ergeben, dadurch gekennzeichnet, dass man als tertiäre alkylaromatische Verbindungen enthaltende Komponente ein solches Produkt verwendet, das als Nebenprodukt bei der Produktion von Cumol erhalten wird, einen Siedebereich von 170–220°C aufweist und 10–80 Gew.-% Diisopropylbenzole sowie 20–90 Gew.-% einer Mischung von $C_4$–$C_9$-Alkylbenzolen enthält, wobei das Dihydroperoxid von p-Diisopropylbenzol bei der Abkühlung der Reaktionsmischung nach der Oxidation selektiv kristallisiert und man es abtrennt und durch saure Katalyse zersetzt, um Hydrochinon und Aceton zu ergeben, während die Monohydroperoxide und Dihydroperoxide von m-Diisopropylbenzol in der oxidierenden Lösung verbleiben und zum Oxidationsreaktor zurückgeführt werden.

2. Verfahren nach Anspruch 1, dadurch gekennzeichnet, dass die tertiäre alkylaromatische Verbindungen enthaltende Komponente 10–80 Gew.-% Diisopropylbenzole und 20–60 Gew.-% einer Mischung von $C_4$–$C_9$-Alkylbenzolen und insbesondere t-Butylbenzol, 1,1-Dimethylpropylbenzol, i- und 1-Hexylbenzole sowie 2- und 3-Phenylhexane enthält.

3. Verfahren nach Anspruch 1 oder 2, dadurch gekennzeichnet, dass das Dihydroperoxid von p-Diisopropylbenzol mittels eines sauren Ionenaustauschharzes zersetzt wird, um Hydrochinon zu ergeben.

4. Verfahren nach einem der Ansprüche 1 bis 3, dadurch gekennzeichnet, dass die Proportion der zum Oxidationsreaktor zurückgeführten oxidierenden Mischung, bezogen auf die Menge des Ausgangsstoffes, im Bereich von 0,01–10:1 und vorzugsweise von 0,05–2:1 liegt.

**Revendications**

1. Un procédé pour produire de l'hydroquinone par oxydation d'une matière de départ mélangée constituée d'un composant contenant un composé (tert-alcoyl)aromatique et un composant contenant des hydroperoxydes produit dans la réaction d'oxydation sélective d'alcoylaromatiques à l'aide d'oxygène à 60–180°C et par séparation du mélange du dihydroperoxyde de p-diisopropylbenzène, qui est décomposé par catalyse acide pour former de l'hydroquinone à 20–150°C, caractérisé en ce que, comme dit composant contenant un composé (tert-alcoyl)aromatique, on utilise un produit obtenu comme sous-produit dans la production du cumène, qui a un intervalle d'ébullition de 170 à 220°C et qui contient 10 à 80% en poids de diisopropylbenzènes et 20 à 90% en poids d'un mélange d'(alcoyl en $C_4$–$C_9$)benzènes, si bien que, par refroidissement du mélange réactionnel après oxydation, le dihydroperoxyde de p-diisopropylbenzène cristallise sélectivement et est séparé et décomposé par catalyse acide pour former de l'hydroquinone et de l'acétone, tandis que les monohydroperoxydes et le dihydroperoxyde de m-diisopropylbenzène demeurent dans la solution oxydante et sont renvoyés dans le réacteur d'oxydation.

2. Procédé selon la revendication 1, caractérisé en ce que ledit composant contenant un composé (tert-alcoyl)aromatique contient 10 à 80% en poids de diisopropylbenzènes et 20 à 60% en poids d'un mélange d'(alcoyl en $C_4$–$C_9$)benzènes, en particulier de tert-butylbenzène, de 1,1-diméthylpropylbenzène, d'iso- et de 1-hexylbenzènes et de 2- et 3-phénylhexanes.

3. Procédé selon la revendication 1 ou 2, caractérisé en ce que l'on décompose le dihydroperoxyde de p-diisopropylbenzène pour qu'il forme de l'hydroquinone à l'aide d'une résine échangeuse d'ions acide.

4. Procédé selon l'une quelconque des revendications 1 à 3, caractérisé en ce que la proportion du mélange oxydant, renvoyée au réacteur d'oxydation, relativement à la quantité de matière de départ, est dans la gamme de 0,01–10/1, de préférence de 0,05–2/1.